Europäisches Patentamt

European Patent Office

Office européen des brevets

(11) Numéro de publication: **0 219 380**
**B1**

## FASCICULE DE BREVET EUROPEEN

(45) Date de publication du fascicule du brevet: **02.05.90**

(51) Int. Cl.⁵: **A 61 F 11/04**

(21) Numéro de dépôt: **86401949.2**

(22) Date de dépôt: **05.09.86**

(54) Appareil de stimulation neurale pour prothèse auditive.

(30) Priorité: **12.09.85 FR 8513528**
**12.09.85 FR 8513529**

(43) Date de publication de la demande:
**22.04.87 Bulletin 87/17**

(45) Mention de la délivrance du brevet:
**02.05.90 Bulletin 90/18**

(84) Etats contractants désignés:
**AT BE CH DE FR GB IT LI LU NL SE**

(56) Documents cités:
**FR-A-2 226 976**
**FR-A-2 465 474**
**GB-A-2 133 697**

(73) Titulaire: **Les Laboratoires MXM, S.A.**
**803 Chemin des Ames du Purgatoire Quartier**
**Croix-Rouge**
**F-06600 Antibes (FR)**

(72) Inventeur: **Weber, Jean-Luc Michel**
**Lotissement Adam 2 Craponne**
**F-13300 Salon de Provence (FR)**
Inventeur: **Bloch, Serge**
**1, rue des Muletiers**
**F-13100 Aix-en-Provence (FR)**
Inventeur: **Noack, Jean-Claude**
**Route de la Malle**
**F-13480 Cabries (FR)**
Inventeur: **Chouard, Claude-Henri**
**10 Boulevard Flandrin**
**F-75016 Paris (FR)**
Inventeur: **Mac Leod, Patrick**
**424 Avenue de la Division Leclerc**
**F-92290 Chatenay-Malabry (FR)**

(74) Mandataire: **Moretti, René et al**
**CABINET BEAU DE LOMENIE 14, rue Raphael**
**F-13008 Marseille (FR)**

Courier Press, Leamington Spa, England.

## Description

L'invention concerne un appareil de stimulation neurale pour prothèse auditive.

Les vibrations mécaniques que constituent les sons, après avoir été transmises aux liquides de l'oreille interne par le jeu du tympan et des osselets, excitent les cellules sensorielles ciliées de la cochlée. Ces cellules ciliées transforment les vibrations mécaniques en signaux électrophysiologiques qu'elles transmettent aux dendrites des fibres du nerf auditif conduisant ces signaux jusqu'au cerveau.

S'agissant de sourds profonds dont les cellules sensorielles ciliées sont déficientes, les appareils d'assistance auditive classiques sont inefficaces car ils ne font qu'augmenter plus ou moins l'énergie mécanique fournie à l'oreille et on doit leur substituer un appareil qui stimule directement le nerf auditif au moyen d'un courant électrique.

Parmi les appareils de stimulation neurale pour prothèse auditive actuellement connus, on distingue ceux dits à "canaux multiples" de ceux du type à "canal unique".

Les premiers reposent sur la constatation qu'une onde acoustique complexe, constituée de nombreuses composantes sinusoïdales de fréquences différentes, donne naissance à des pics de vibration répartis sur la membrane basilaire qui, à l'intérieur de la cochlée, porte les cellules ciliées. Cette membrane vibre avec une amplitude maximale au point qui est en résonance mécanique avec la fréquence du son, les hautes fréquences faisant vibrer la base de la spirale cochléaire et les basses fréquences son sommet. Les appareils de stimulation à canaux multiples exploitent cette propriété au moyen d'un système à $n$ électrodes implantées dans la cochlée, chaque électrode étant affectée à l'un de $n$ groupes consécutifs de fibres du nerf auditif. Ces électrodes sont excitées sélectivement par des canaux indépendants auxquels sont affectées certaines bandes de fréquence, de sorte que l'ensemble de ces canaux indépendants permet de couvrir une zone prédéterminée du spectre audible.

Ces appareils de stimulation du nerf cochléaire à canaux multiples, dont on trouvera un exemple dans le brevet FR—A—2 383 657, sont performants mais d'un prix de revient élevé en raison de leur sophistication. De plus, la mise en place du faisceau de $n$ électrodes à l'intérieur de la cochlée est une opération très délicate qui doit être faite une fois pour toutes en raison des risques de détérioration de certains tissus internes très fragiles de la cochlée que présesenterait sa répétition. C'est la raison pour laquelle on réserve la mise en place de ces appareils à canaux multiples, onéreux mais efficaces, à des patients dont les paramètres électrophysiologiques laissent penser qu'ils on suffisamment de restes auditifs pour en exploiter pleinement les possibilités.

Lorsque les patients ne sont pas capables d'utiliser la richesse de l'information spectrale apportée par les appareils à canaux multiples, on préfère généralement recourir aux appareils à canal unique qui sont beaucoup moins coûteux et délicats à implanter. Ces appareils comprennent une électrode de stimulation unique, disposée soit au voisinage de la base de la cochlée, soit à l'extérieur de celle-ci, dans l'oreille moyenne. Comme ceux à canaux multiples, des appareils à canal unique comprennent un microphone apte à capter un signal sonore, un processeur électronique qui convertit le signal sonore en signaux électriques et des moyens de transmission de ces signaux électriques à l'électrode de stimulation du nerf auditif.

Un appareil de stimulation du nerf auditif à canal unique est notamment décrit dans la demande de brevet EP—A—0076069. Un tel appareil permet à des malades dont la surdité est soit congénitale, soit acquise à l'âge adulte, de percevoir le rythme et l'intensité du son, mais il ne fournit pas suffisamment d'informations au système nerveux pour permettre à un sujet sourd d'identifier son interlocuteur dans une conversation à plusieurs personnes sans recourir à la lecture labiale.

On connaît également par le brevet GB—A—2 133 697 un appareil à canal unique comprenant un transducteur apte à capter un signal sonore, un filtre passe-bas, un amplificateur logarithmique destiné à effectuer une compression du signal filtré provenant du transducteur et un détecteur de crête permettant d'extraire la fréquence fondamentale d'un signal de parole, c'est-à-dire le voisement.

Grâce à l'extraction du voisement, cet appareil permet de restituer à un patient qui en est équipé la "coloration" du signal sonore émis par le locuteur.

Toutefois, il présente un grace défaut en ce sens que le signal de stimulation est constitué d'impulsions dont la durée est sensiblement égale à la moitié de la période fondamentale du signal de parole. Autrement dit, plus la voix du locuteur est basse, plus le son perçu par le patient est intense, et inversement, ce qui ne correspond pas à la réalité des phénomènes physiques perçus par les sujets dotés d'une ouïe normale.

En effet, si plusieurs locuteurs sont placés à des distances différentes d'un patient, ce dernier risque d'être induit en erreur puisque le son perçu le plus fort ne proviendra pas nécessairement du locuteur le plus proche de lui.

Par ailleurs, cet appareil comprend des moyens relativement complexes destinés à opérer une distinction entre signaux voisés et signaux non voisés. Lorsqu'un son non voisé est détecté, une stimulation de caractéristiques prédéterminées est appliquée au patient. Il en ira de même si, pour une raison quelconque, l'appareil n'a pas détecté un son voisé et l'a identifié comme non voisé. Dans les deux cas, l'information transmise au patient est donc arbitraire.

L'invention a pour but d'améliorer l'aptitude conversationnelle des sourds profonds au moyen d'un appareil à canal unique de construction simple qui fournit au système nerveux du patient une information plus élaborée que celle à laquelle ils ont accès les appareils à canal unique conventionnels.

A cet effet, l'invention a pour objet un appareil de stimulation neurale pour prothèse auditive à canal unique comprenant un microphone apte à capter un signal sonore, un processeur électronique qui convertit ce signal sonore en un signal électrique et comprend des circuits permettant d'extraire d'un signal de parole d'un locuteur une composante représentative du voisement et de produire un signal électrique de même fréquence que ladite compostante, et des moyens de transmission dudit signal électrique à un circuit d'excitation d'une électrode de stimulation d'un nerf auditif, caractérisé en ce que le processeur électronique comprend des circuits de modulation et/ou de codage dudit signal électrique en fonction de l'énergie totale du signal sonore capté pour l'application à l'électrode de stimulation, par le circuit d'excitation, d'un signal de stimulation représentatif tant de l'énergie totale que d'un possible voisement du signal sonore.

De préférence, le circuit d'excitation comprend une source de courant émettant des impulsions de stimulation à courant constant ayant une charge électrique prédéterminée fonction de l'énergie totale du signal sonore et des moyens pour engendrer consécutivement à chaque impulsion de stimulation d'une polarité donnée une impulsion de neutralisation de réactions électrochimiques ayant une polarité opposée et la même charge électrique que l'impulsion de stimulation précédente.

Cette forme préférée de réalisation permet de rendre reversibles et inoffensives les réactions électrochimiques complexes engendrées au niveau des tissus biologiques par la stimulation électrique et qui, avec les appareils classiques, sont susceptibles de modifier les caractéristiques électriques du stimulus.

L'invention s'applique en particulier au traitement des surdités profondes et des bourdonnement d'oreilles.

D'autres caractéristiques et avantages de l'invention apparaîtront plus clairement à la lecture de la description qui suit, faite, à titre d'exemple non limitatif, en se reportant aux dessins annexés sur lesquels:

La figure 1 est un schéma-bloc d'un premier mode de réalisation de l'appareil de stimulation neurale.

La figure 2 est un graphique montrant la forme du signal de commande et du courant envoyé à l'électrode de stimulation de l'appareil de la figure 1.

La figure 3 est un schéma électrique équivalent d'une partie du circuit d'excitation de l'appareil pendant les phases de stimulation.

La figure 4 est un schéma équivalent d'une partie du circuit d'excitation de l'appareil pendant les phases de neutralisation.

La figure 5 est un schéma électrique d'une variante du circuit d'excitation de l'appareil de la figure 1.

La figure 6 est un graphique analogue à celui de la figure 2 montrant la forme de courant obtenu avec le circuit d'excitation de la figure 5.

La figure 7 est un schéma-bloc d'un second mode de réalisation de l'appareil de stimulation neurale.

La figure 8 est un graphique montrant la forme du signal de commande et du courant impulsionnel produit par le circuit d'excitation de l'appareil de la figure 7, et

La figure 9 est un graphique montrant des formes de signaux illustrant une variante de réalisation de l'appareil de la figure 7.

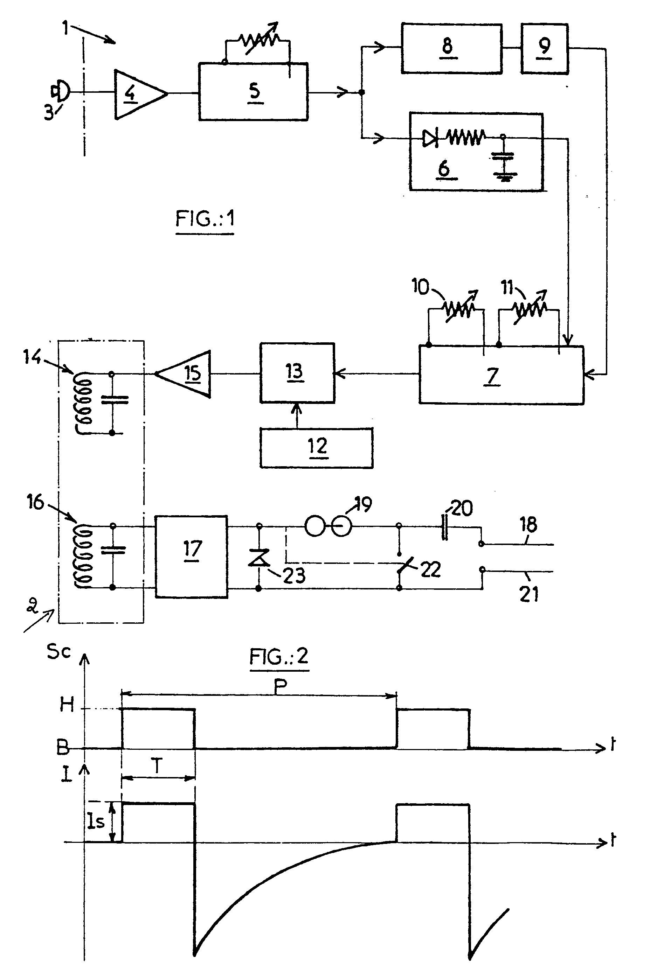

On a représenté à la figure 1 les circuits constituant le sous-ensemble émetteur 1 et le sous-ensemble récepteur 2 de l'appareil.

Le sous-ensemble émetteur 1 comprend un microphone 3 qui est destiné à capter un signal sonore et dont la sortie attaque un amplificateur 4 à gain réglable entre, par exemple, 30 et 1000. La sortie de l'amplificateur à gain variable 4 est appliquée à l'entrée d'un compresseur 5 dont le rôle est d'adapter la dynamique du signal d'information sonore à la dynamique de l'oreille. C'est ainsi, par exemple, qu'un bruit variant entre 40 dBA (seuil de perception) et 100 dBA (seuil douloureux) pourra être comprimé dans le circuit 5 pour obtenir un écart de 6 dB pour l'énergie du signal électrique de stimulation, cette compression étant évidemment proportionnelle à chaque instant à l'énergie instantanée du son recueilli de façon à ne pas déformer l'échelle des sensations dans le cerveau (une telle compression étant effectuée par le tympan et l'oreille moyenne chez les personnes dont l'ouïe est normale). Le compresseur 5 peut être constitué par exemple par un circuit électronique à fonction de transfert logarithmique. La sortie du compresseur 5 est appliquée à l'entrée d'un étage 6 de redressement et de filtrage du signal compressé produisant une tension représentative de l'énergie compressée du signal sonore original pour piloter un générateur d'impulsions 7. La sortie du compresseur 5 est également appliquée à l'entrée d'un filtre passe-bas 8, tel qu'un filtre du troisième ordre ayant une fréquence de coupure de quelques centaines de Hertz, par exemple 400 Hertz environ. Le signal de sortie du filtre 8 est couplé à l'entrée d'un détecteur 9 dont le seuil est réglable, de manière à ne prendre en compte que des signaux représentatifs d'un signal sonore ayant un niveau minimal de, par exemple, 40 dBA. On notera, cependant, que le détecteur 9 pourrait aussi bien être placé en amont du filtre 8 ou du compresseur 5.

Le signal électrique ayant traversé le filtre 8 et le détecteur 9 correspond à la fréquence fondamentale du signal sonore appliqué au microphone 3, c'est-à-dire que, dans le cas d'un signal de parole, il est représentatif du voisement de la parole du locuteur. Ce signal de voisement attaque l'entrée de déclenchement du générateur d'impulsions 7 qui produit, en synchronisme avec le signal de voisement, des impulsions rectangulaires dont la largeur est fonction du signal de volume sonore issu de l'étage 6 de redressement et de filtrage. Ce générateur d'impulsions comporte deux potentiomètres 10 et 11 dont l'un 10 permet à l'équipe médicale, lors de l'implantation de l'appareil sur un patient, de régler la durée maximale des impulsions émises par le générateur 7 et dont l'autre 11 offre au patient une possibilité de réglage de "volume" en cours d'utilisation. Le patient peut ainsi, de lui-même, diminuer la durée maximale des impulsions jusqu'à, par exemple, le tiers de la durée maximale imposée par l'équipe médicale.

Le signal de sortie Sc du générateur d'impulsions 7 est représenté à la figure 2 où T représente la durée des impulsions et P la période du signal voisé. De préférence, les réglages effectués au moyen du potentiomètre 10 sont tels que T est toujours compris entre 5 et 500 µs environ et varie entre deux extrêmes suivant une loi logarithmique pour des niveaux acoustiques allant de 40 dBA à 80 dBA environ. Un signal haute-fréquence, de par example 3 MHz, émis par un oscillateur 12 est modulé, par le train d'impulsions délivré par le générateur 7, dans un modulateur 13. Le signal résultant attaque une antenne émettrice 14 par l'intermédiaire d'un étage de puissance 15.

Le signal émis par l'antenne 14 est reçu par le sous-ensemble récepteur 2 au moyen d'une antenne réceptrice 16 accordée sur la fréquence de la porteuse délivrée par l'oscillateur 12. L'antenne réceptrice 16 est suivie d'un étage de démodulation 17 qui assure le redressement et le filtrage du signal reçu et restitue un signal impulsionnel conforme à celui de la figure 2. L'une des bornes de sortie de l'étage 17 est connectée à l'électrode de stimulation 18 par l'intermédiaire d'une source de courant constant 19 et d'un condensateur 20, tandis que son autre borne est connectée à l'électrode de masse 21 associée à l'électrode de stimulation 18. Un interrupteur électronique 22 commandé par le signal de sortie de l'étage 17 est connecté entre, d'une part, le point commun à la source de courant 19 et le condensateur 20 et, d'autre part, l'électrode de masse 21. Cet interrupteur électronique 22, qui peut être constitué par exemple par un montage classique à deux transistors, est ouvert lorsque le signal de sortie de l'étage 17 (voir figure 2) est à son niveau haut et que la source de courant 19 excite l'électrode stimulatrice 18. Il se ferme quand le signal passe à son niveau bas, permettant ainsi la décharge du condensateur 20 à travers le circuit comprenant l'interrupteur électronique 22 et l'espace inter-électrodes. Enfin, une diode de Zener est connectée entre les bornes de sortie de l'étage 17 pour limiter à une valeur admissible, par exemple 20 volts, les surtensions éventuelles pouvant apparaître entre ces bornes.

De manière conventionnelle, le sous-ensemble récepteur 2 formant circuit d'excitation est destiné à être implanté entre le peau et la boîte crânienne d'un patient, au voisinage d'une oreille de ce dernier. L'électrode de stimulation 18 peut être localisée, soit dans la cochlée, soit à l'extérieur de celle-ci, pourvu que la cochlée se trouve comprise dans le champ électrique induit par l'électrode active et l'électrode de masse. Le sous-ensemble émetteur extérieur 1 est porté par le patient de manière que l'antenne émettrice se trouve placée en regard de l'antenne réceptrice. Le microphone 3 est, de préférence, fixé avec l'antenne émettrice 14, soit sur un moulage intra-auriculaire, soit sur un contour d'oreille. Ces deux éléments sont reliés par un cordon électrique à un boîtier porté par le patient dans l'une de ses poches et contenant les autres circuits du sous-ensemble émetteur.

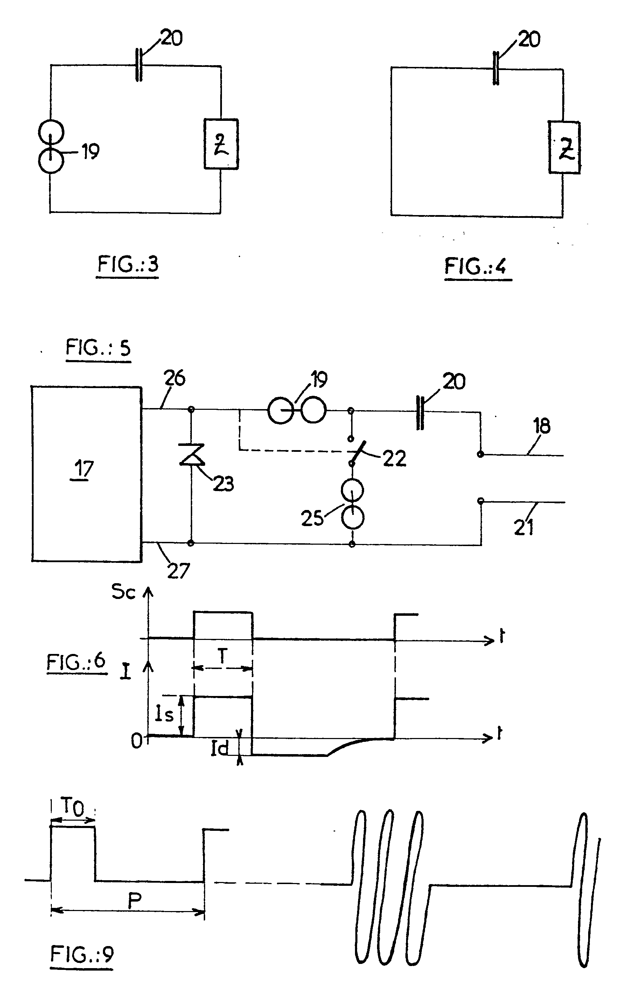

En fonctionnement, un signal de parole capté par le microphone 3 est traité par les étages 4 à 9 qui délivrent en sortie du générateur d'impulsions 7 le signal Sc de la figure 2. Après modulations, amplification, transmission et démodulation, ce même signal Sc pilote la source de courant constant 19. Lorsque le signal Sc passe à son niveau haut H, la source de courant 19 est rendue conductrice, tandis que l'interrupteur électronique 22 est ouvert. Le schéma électrique équivalent du circuit d'excitation est alors celui représenté à la figure 3 où la source de courant 19 débite un courant constant dans une impédance Z, symbolisant l'impédance des tissus biologiques situés entre les électrodes 18 et 21, par l'intermédiaire du condensateur 20. Comme le montre le graphique de la figure 2 où la deuxième courbe I représente l'allure du courant dans l'électrode de stimulation 18, celle-ci est alimentée par un courant constant d'intensité prédéterminée Is pendant la durée T où le signal de commande est à son niveau haut. Quand ce dernier passe à son niveau bas B, il bloque la source de courant 19 et ferme l'interrupteur 22. Le schéma électrique équivalent du circuit d'excitation est alors celui représenté à la figure 4 où le condensateur 20 se trouve connecté en série avec l'impédance Z par l'intermédiaire des électrodes 18 et 21. Au moment de la fermeture de l'interrupteur 22, le condensateur 20 se décharge dans l'impédance Z et il se produit donc une inversion de sens du courant comme représenté à la figure 2, jusqu'à l'apparition d'une nouvelle impulsion de signal de commande Sc qui provoque un nouveau cycle de conduction de la source de courant 19, de charge du condensateur 20, puis de décharge de celui-ci consécutivement à la fermeture de l'interrupteur 22.

Il résulte de ce qui précède que l'on envoie dans les tissus biologiques à stimuler un courant constant d'intensité prédéterminée Is pendant un temps T proportionnel à la grandeur de commande, ce qui correspond à une charge $Q = Is \times T$ dans le condensateur 20 et dans les tissus. Ce mode de stimulation à courant constant régulé permet de s'affranchir tant des différences d'impédance que peuvent présenter les tissus biologiques d'un individu à l'autre, que des dérives d'impédance avec le temps des circuits électriques, dérives qui peuvent être dues, par exemple, à des modifications de la position ou des caractéristiques électriques des électrodes, à un vieillissement des composants électroniques de l'implant, à des changements de position de l'antenne émettrice par rapport à l'antenne réceptrice, etc. En outre, comme le condensateur se décharge consécutivement à chaque phase ou alternance de charge, il apparaît que la valeur moyenne du courant dans l'électrode de stimulation est nulle sur une période complète ou, autrement dit, que le bilan des charges électriques envoyées au nerf auditif est parfaitement équilibré sur une période complète, ce qui présente une grande importance d'un point de vue physiologique.

Un autre avantage de l'appareil décrit ci-dessus réside dans le mode de traitement du signal sonore qui permet, à partir d'un signal de parole, d'exciter le nerf auditif d'un patient par un signal électrophysiologique représentatif tant de l'amplitude que du voisement de la parole. En d'autre termes, ce signal électrophysiologique est débarrassé des informations liées à l'élocution proprement dite, tout en conservant le voisement qui est très caractéristique d'un individu. On peut donc admettre que, moyennant certains apprentissages, l'appareil décrit ci-dessus devrait permettre à un sourd profond de déterminer celui des interlocuteurs qui est en train de parler, non par vision du mouvement de ses lèvres, mais par reconnaissance directe de son voisement. Bien entendu, cette faculté n'affranchira pas le malade de la nécessité de recourir à la lecture labiale pour donner un contenu intelligible aux paroles prononcées, mais elle constitue un progrès significatif par rapport aux appareils à canal unique de la technique antérieure qui ne donnaient à percevoir aux malades qu'un bruit complexe modulé en amplitude difficilement identifiable.

Le variante d'exécution du circuit d'excitation de la figure 5 diffère de celle de la figure 1 uniquement par la présence d'un régulateur de courant de décharge 25 connecté en série entre l'interrupteur électronique 22 et l'électrode de masse 21. Ce régulateur permet de limiter la valeur maximale du courant dans l'électrode de masse 21 à une valeur Id pendant la phase de décharge du condensateur 20 comme représenté sur la partie négative de la courbe I de la figure 6. Le circuit d'excitation de la figure 5 est, par ailleurs, en tous points identique à celui de la figure 1, la diode de Zener 23 facultative assurant comme dans ce dernier une protection des tissus contre les surtensions éventuelles pouvant apparaître entre les bornes 26 et 27.

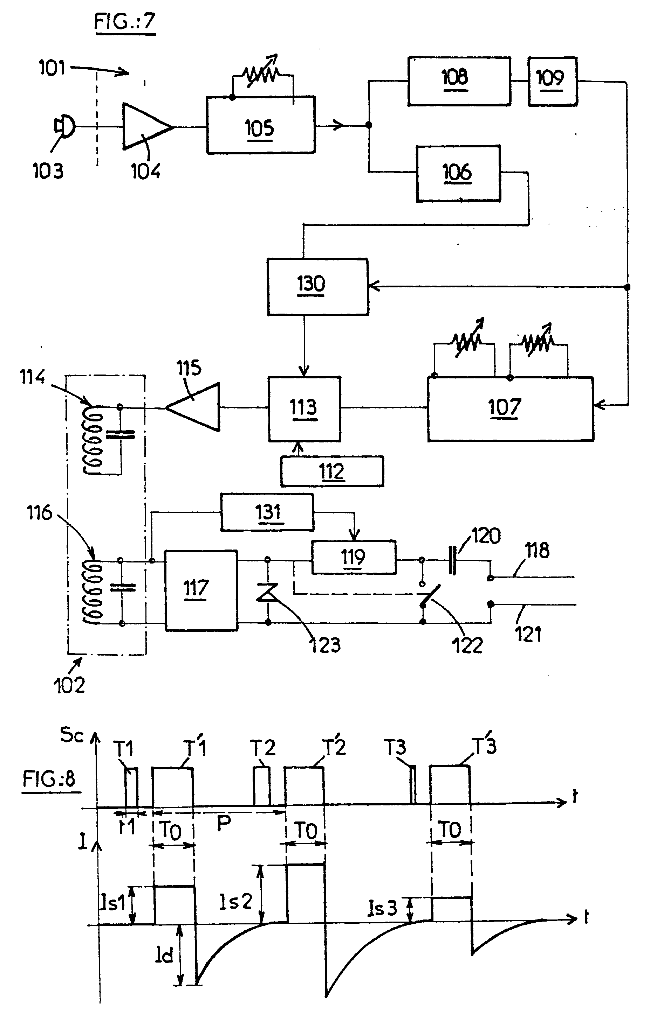

On se reportera maintenant à la figure 7, où les mêmes références qu'à la figure 1, mais augmentées du nombre 100, ont été utilisées pour repérer les mêmes composants. Ce second mode de réalisation diffère de celui de la figure 1 uniquement en ce que concerne le mode de codage du volume du signal sonore capté. C'est ainsi que l'étage 106 de redressement et de filtrage attaque, non pas le générateur d'impulsions 107, mais le modulateur 113 par l'intermédiaire d'un étage 130 de codage de l'amplitude du courant.

Le signal impulsionnel résultant de ce codage est représenté sous forme démodulée à la figure 8. Il comprend une première impulsion T1 de codage de courant dont la durée t1, variable par exemple de 1 à 5 µs, est proportionnelle à l'intensité du courant qui doit être généré par la source 119, et une seconde impulsion T'1, de durée T0 constante dans cette application, pendant laquelle le courant dont l'intensité est déterminée par l'impulsion T1 est envoyé à l'électrode 118. L'étage de codage 130 fonctionne de manière similaire au générateur d'impulsions 7 et émet l'impulsion T1 de durée t1 proportionnelle au volume sonore sous la commande du signal de voisement appliqué à son entrée de déclenchement. Le générateur d'impulsions 107 émet l'impulsion T'2 de durée constante T0 avec un retard prédéterminé par rapport au front montant de l'impulsion T'1. Par conséquent, le temps P séparant les fonts montants de deux impulsions T'1, T'2, T'3 consécutives représente, comme dans le premier mode de réalisation, la période fondamentale du signal de parole. Après modulation, ce signal est transmis à l'antenne réceptrice 116.

Un étage de décodage 131 connecté à celle-ci commande le générateur de courant 119 pour qu'il génère un courant d'intensité Is1, Is2, Is3 proportionnelle à la durée de l'impulsion correspondante T1, T2, T3. Ce courant constant proportionnel au volume du signal sonore capté est envoyé à l'électrode 118 pendant la durée T0, constante dans cette application, de l'impulsion T'1, T'2, T'3. Pour le reste, le fonctionnement de ce second mode de réalisation est parfaitement identique à celui de la figure 1. Il apparaît que ce second mode de réalisation permet également d'envoyer dans le nerf auditif une quantité d'électricité parfaitement maîtrisée.

On a également représenté pour mémoire, à la figure 9, dex signaux illustrant une variante d'exécution du second mode de réalisation. Dans cette variante, le générateur d'impulsions 107 émet des impulsions de durée T0 dont la période de récurrence T est celle du fondamentale du signal sonore capté. L'étage de codage 130 détermine en fonction de l'intensité du courant à générer l'amplitude de la porteuse modulée par le signal de sortie du générateur 107. Au niveau du sous-ensemble émetteur 102, l'étage de décodage 131 pilote le générateur de courant 119 pour produire un courant proportionnel à l'amplitude de la porteuse qui lui est appliquée.

Bien entendu, il est encore possible de recourir à d'autres moyens tels que, par exemple, la modulation de fréquence ou la modulation de phase, pour générer un courant proportionnel au volume du signal sonore qui sera envoyé à l'électrode de stimulation pendant un temps constant prédéterminé. Ces différentes solutions ont en commun de permettre d'envoyer au nerf auditif une quantité d'électricité parfaitement déterminée.

Cependant, il doit être compris que l'invention n'est nullement limitée à la mise en oeuvre de cette caractéristique avantageuse et que le signal électrophysiologique représentatif du voisement et du volume du signal de parole capté pourrait également avoir une forme sinusoïdale ou autre, variable, non pas en intensité ou en largeur d'impulsion, mais par exemple en tension.

## Revendications

1. Appareil de stimulation neurale pour prothèse auditive à canal unique comprenant un microphone (3) apte à capter un signal sonore, un processeur électronique (4—9; 104—109) qui convertit ce signal sonore en un signal électrique de commande et comprend des circuits (7—9; 107—109) permettant d'extraire d'un signal de parole d'un locuteur une composante représentative du voisement et de produire un signal électrique ayant la même fréquence que ladite composante, et des moyens (12—17; 112—117) de transmission du signal électrique de commande à un circuit (18—23; 118—123; 131) d'excitation d'une électrode (18; 118) de stimulation d'un nerf auditif, caractérisé en ce que le processeur électronique comprend des circuits (6, 7; 106, 113, 130) de modulation et/ou de codage dudit signal électrique en fonction de l'énergie totale du signal sonore capté pour produire un signal électrique de commande (Sc) assurant l'application à l'électrode de stimulation, par le circuit d'excitation (18—23; 118—123, 131), d'un signal de stimulation (I) représentatif tant de l'énergie totale que d'un possible voisement du signal sonore.

2. Appareil selon la revendication 1, caractérisé en ce que le circuit d'excitation comprend une source de courant (19; 119) émettant des impulsions de stimulation à courant constant ayant une charge électrique prédéterminée fonction de l'énergie totale du signal sonore et des moyens (20, 22; 120, 122) pour engendrer consécutivement à chaque impulsion de stimulation d'une polarité donnée une impulsion de neutralisation de réactions électrochimiques ayant une polarité opposée et la même charge électrique que l'impulsion de stimulation précédente.

3. Appareil selon la revendication 2, caractérisé en ce que la source de courant (19; 119) est connectée à l'électrode de stimulation (18; 118) par l'intermédiaire d'un condensateur (20; 120) et en ce qu'il est prévu un interrupteur électronique (22; 122) commandé par lesdits moyens de transmission (12—17; 112—117) pour connecter le condensateur (20; 120) en série entre l'électrode de stimulation (18; 118) et une électrode de masse (21; 121) consécutivement à chacune desdites impulsions.

4. Dispositif selon la revendication 3, caractérisé en ce qu'il comprend un régulateur (25) de courant de décharge du condensateur (20) connecté en série entre l'interrupteur électronique (22) et l'autre électrode (21).

5. Dispositif selon la revendication 3, caractérisé en ce que la source de courant (19, 119) et le condensateur (20; 120) sont connectés en série avec l'électrode de stimulation (18; 118) et l'interrupteur électronique (22, 122) est connecté entre, d'une part, le point commun à la source de courant et au condensateur et, d'autre part, l'électrode de masse (21; 121).

6. Dispositif selon l'une quelconque des revendications 3 à 5, caractérisé en ce que le signal électrique de commande (Sc) est un signal rectangulaire qui rend conductrice la source de courant (19; 119) et ouvre l'interrupteur (22; 122) lorsqu'il est à un premier niveau et qui bloque la source de courant et ferme l'interrupteur lorsqu'il se trouve à un deuxième niveau.

7. Appareil selon l'une quelconque des revendications 1 à 6, caractérisé en ce que le circuit d'extraction de ladite composante représentative du voisement (8; 108) est un filtre passe-bas.

8. Appareil selon la revendication 7, caractérisé en ce que le processeur électronique comprend un amplificateur à gain variable (4; 104) et un compresseur (5; 105) dont la sortie est connectée respectivement au filtre passe-bas (8; 108) et à un étage (6; 106) de redressement et de filtrage produisant un signal de sortie représentatif de l'énergie compressée du signal sonore capté.

9. Appareil selon la revendication 8, caractérisé en ce que les sorties du filtre passe-bas (8) et de l'étage de redressement et de filtrage (6) sont connectées à un générateur (7) délivrant des impulsions de durée modulée (T) proportionnelle à ladite énergie, avec une période de récurrence (P) égale à celle de ladite composante.

10. Appareil selon la revendication 9, caractérisé en ce que le circuit d'excitation (18—23) envoie à l'électrode de stimulation un courant d'intensité constante prédéterminée pendant la durée modulée (T) des impulsions.

11. Appareil selon la revendication 8, caractérisé en ce que la sortie du filtre passe-bas (108) est connectée à un générateur (107) appliquant des impulsions (T'1, T'2, T'3) de même fréquence que ladite composante à un étage (130) de codage de l'intensité du courant du signal de stimulation en fonction du signal de sortie de l'étage de redressement et de filtrage (106), l'étage de codage délivrant ledit signal de commande (Sc).

12. Appareil selon la revendication 11, caractérisé en ce que le circuit d'excitation (118—123, 131) comprend un étage (131) de décodage du signal de commande (Sc) qui détermine l'intensité du courant (Is1, Is2, Is3) fourni à l'électrode de stimulation (18) pendant la durée (T0) desdites impulsions (T'1, T'2, T'3).

13. Appareil selon l'une quelconque des revendications 11 et 12, caractérisé en ce que lesdites impulsions T'1, T'2, T'3 ont une durée (T0) constante.

14. Appareil selon l'une quelconque des revendications 1 à 13, caractérisé en ce que le circuit d'excitation (18—23; 118—123; 131) et l'électrode de stimulation (18; 118) forment un premier sous-ensemble (2) implantable sous la peau d'un patient, le microphone (3) et le processeur électronique (4—9; 104—109) forment un second sous-ensemble (1) adapté pour être porté extérieurement par le patient, et les moyens de transmission (12—17; 112—117) comprennent des antennes réceptrice (16) et émettrice (14) accordées, associées respectivement aux premier et second sous-ensembles.

**Patentansprüche**

1. Apparat zur neuralen Stimulation für Einkanal-Hörprothesen, umfassend ein Mikrofon (3) zur Aufnahme eines Tonsignals, einen elektronischen Prozessor (4—9; 104—109), der dieses Tonsignal in ein elektrisches Steuersignal umwandelt und Schaltkreise (7—9; 107—109) umfaßt, die die Heraustrennung einer für den Stimmcharakter repräsentativen Komponente aus einem Sprechsignal eines Redners und die Erzeugung eines elektrischen Signals derselben Frequenz die besagte Komponente gestattet, und Mittel (12—17; 112—117) zur Übertragung des elektrischen Steuersignals zu einem Schaltkreis (18—23; 118—123, 131) zur Erregung einer Elektrode (18; 118) für die Stimulation eines Hörnervs, dadurch gekennzeichnet, daß der elektronische Prozessor Schaltkreise (6, 7; 106, 113, 130) zur Modulation und/oder Kodierung des elektrischen Signals als Funktion der Gesamtenergie des aufgenommenen Tonsignals zwecks Erzeugung eines elektrischen Steuersignals (Sc), das die Aufbringung eines Stimulationssignals (I), welches sowohl die Gesamtenergie als auch einen möglichen Stimmcharakter des Tonsignals repräsentiert, auf die Stimulationselektrode über den Erregerkreis (18—23; 118—123, 131) gewährleistet, umfaßt.

2. Apparat nach Anspruch 1, dadurch gekennzeichnet, daß der Erregerkreis eine Stromquelle (19; 119), die Gleichstrom-Stimulationsimpulse mit einer als Funktion der Gesamtenergie des Tonsignals vorbestimmten elektrischen Ladunt entsendet, und Mittel (20, 22; 120, 122) zur Erzeugung eines Impulses zur Neutralisierung von elektrochemischen Reaktionen mit entgegengesetzter Polarität und derselben elektrischen Ladung wie der vorhergehende Stimulationsimpuls im Anschluß an jeden Stimulationsimpuls einer gegebenen Polarität umfaßt.

3. Apparat nach Anspruch 2, dadurch gekennzeichnet, daß die Stromquelle (19; 119) über einen Kondensateor (20; 120) an die Stimulationselektrode (18; 118) angeschlossen ist, und daß ein elektronischer Unterbrecher (22; 122) vorgesehen ist, der von den Übertragungsmitteln (12—17; 112—117) gesteuert wird, um den Kondensator (20; 120) zwischen der Stimultationselektrode (18; 118) und einer Massenelektrode (21; 121) im Anschluß an jeden Impuls in Serie zu schalten.

4. Vorrichtung nach Anspruch 3, dadurch gekennzeichnet, daß sie einen Regulator (25) für den Entladungsstrom des Kondensators (20) umfaßt, welcher zwischen dem elektronischen Unterbrecher (22) und der anderen Elektrode (21) in Serie geschaltet ist.

5. Vorrichtung nach Anspruch 3, dadurch gekennzeichnet, daß die Stromquelle (19, 119) und der Kondensator (20; 120) mit der Stimulationselektrode (18; 118) in Serie geschaltet sind und der elektronische Unterbrecher (22, 122) zwischen dem der Stromquelle und dem Kondensator gemeinsamen Punkt einerseits und der Massenelektrode (21; 121) anderseits zwischengeschaltet ist.

6. Vorrichtung nach einem der Ansprüche 3 bis 5, dadurch gekennzeichnet, daß das elektrische Steuersignal (Sc) ein Rechtecksignal ist, welches die Stromquelle (19; 119) leitend macht und dem Unterbrecher (22; 122) öffnet, wenn es sich in einem ersten Niveau befindet, und welches die Stromquelle sperrt und den Unterbrecher schließt, wenn es sich in einem zweiten Niveau befindet.

7. Apparat nach einem der Ansprüche 1 bis 6, dadurch gekennzeichnet, daß der Schaltkreis zur Heraustrennung der für den Stimmcharakter repräsentativen Komponente (8; 108) ein Tiefpaßfilter ist.

8. Apparat nach Anspruch 7, dadurch gekennzeichnet, daß der elektronische Prozessor einen Verstärker mit variablem Verstärkungsgrad (4; 104) und einen Verdichter (5; 105) umfaßt, dessen Ausgang an den Tiefpaßfilter (8; 108) bzw, an eine Gleichrichter- und Filterstufe (6; 106), die ein für die verdichtete Energie des aufgenommenen Tonsignals repräsentatives Signal erzeugt, angeschlossen ist.

9. Apparat nach Anspruch 8, dadurch gekennzeichnet, daß die Ausgänge des Tiefpaßfilters (8) und der Gleichrichter- und Filterstufe (6) mit einem Generator (7) verbunden sind, der Impulse von zur Energie proportionaler Modulationsdauer (T) mit einer Wiederholungsperiode (P) gleich jener der Komponente abgibt.

10. Apparat nach Anspruch 9, dadurch gekennzeichnet, daß der Erregerkreis (18—23) während der Modulationsdauer (T) der Impulse Strom von einer bestimmten konstanten Stärke an die Stimulationselektrode sendet.

11. Apparat nach Anspruch 8, dadurch gekennzeichnet, daß der Ausgang des Tiefpaßfilters (108) mit einem Generator (107) verbunden ist, der Impulse (T'1, T'2, T'3) derselben Frequenz wie die Komponente an eine Kodierstufe (130) der Stromstärke des Stimulationssignals als Funktion des Ausgangssignals der Gleichrichter- und Filterstufe (106) anlegt, wobei die Kodierstufe das Steuersignal (Sc) abgibt.

12. Apparat nach Anspruch 11, dadurch gekennzeichnet, daß der Erregerkreis (118—123, 131) eine Dekodierstufe (131) des Steuersignals (Sc) umfaßt, welche die Stärke des an die Stimulationselektrode (118) während der Dauer (T0) des Impulse T'1, T'2, T'3) gelieferten Stroms (Is1, Is2, Is3) bestimmt.

13. Apparat nach einem der Ansprüche 11 und 12, dadurch gekennzeichnet, daß die Impulse (T'1, T'2, T'3) konstante Dauer (T0) haben.

14. Apparat nach einem der Ansprüche 1 bis 13, dadurch gekennzeichnet, daß der Erregerkreis (18—23; 118—123; 131) und die Stimulationselektrode (18; 118) eine erste Untereinheit (2) bilden, die unter der Haut eines Patienten implantierbar ist, das Mikrofon (3) und der elektronische Prozessor (4—9; 104—109) eine zweite Untereinheit (1) bilden, die vom Patienten außen getragen werden kann, und die Übertragungsmittel (12—17; 112—117) aufeinander abgestimmte Empfangs- (16) und Sendeantennen (14) umfassen, die der ersten bzw. der zweiten Untereinheit zugeordnet sind.

## Claims

1. A single-channel nerve-stimulating device for auditory prosthesis comprising a microphone (3) designed to pick up a sound signal, an electronic processor (4—9; 104—109) which converts this sound signal into an electrical control signal (Sc), an electrode (18; 118) for stimulating an auditory nerve, an exciting circuit (18—23; 118—123, 131) for applying to the said electrode a stimulation signal (I) and means (12—17; 112—117) of transmitting the said electrical control signal to the exciting circuit, the said electronic processor comprising circuits (7—9; 107—109) making it possible to extract from a speech signal of a speaker a component representing the voicing and to generate an electrical signal having the same frequency as the said component, characterized in circuits (6, 7; 106, 113, 130) of the said electronic processor for modulating and/or coding the said electrical signal as a function of the total energy of the sound signal picked up, in order to generate a control signal (Sc) and, through the exciting circuit (18—23; 118—123, 131) a stimulation signal (I) which is a function of the control signal, and which represent both the total energy and a possible voicing the sound signal.

2. A device as claimed in claim 1, characterized in that the exciting circuit comprises a current source (19; 119) emitting constant-current stimulation pulses having a predetermined electrical charge as a function of the total energy of the sound signal, and means (20, 22; 120, 122) of generating, following each stimulation pulse of a given polarity, a pulse neutralizing electrochemical reactions which has an opposite polarity and the same electrical charge as the preceding stimulation pulse.

3. A device as claimed in claim 2, characterized in that the current source (19; 119) is connected to the stimulation electrode (18—118) by means of a capacitor (20; 120), and wherein there is an electronic switch (22; 122) controlled by the said transmission means (12—17; 112—117) in order to connect the capacitor (20; 120) in series between the stimulation electrode (18—118) and a shell electrode (21; 121) following each of the said pulses.

4. A device as claimed in claim 3, characterized in that regulator (25) of the discharge current of the capacitor (20) connected in series between the electronic switch (22) and the other electrode (21).

5. A device as claimed in claim 3, characterized in that the current source (19; 119) and the capacitor (20; 120) are connected in series to the stimulation electrode (18; 118), and the electronic switch (22; 122) is connected between, on the one hand, the point common to the current source and to the capacitor and, on the other hand, the shell electrode (21, 121).

6. A device as claimed in either one of claim 3 to 5, characterized in that the electrical control signal (Sc) is a rectangular signal which makes the current source (19; 119) conductive and which opens the switch (22, 122) when it is at a first level and blocks the current source and closes the switch when it is at a second level.

7. A device as claimed in either one of claims 1 to 6, characterized in that the circuit for extracting the said component (8—108) representing the voicing is a low-pass filter.

8. A device as claimed in claim 7, characterized in that the electronic processor comprises a variable-gain amplifier (4; 104) and a compressor (5; 105), the output of which is connected respectively to the low-pass filter (8; 108) and to a rectifying and filtering stage (6; 106) generating and output signal representing the compressed energy of the sound signal picked up.

9. A device as claimed in claim 8, characterized in that the outputs of the low-pass filter (8) and of the rectifying and filtering stage (6) are connected to a generator (7) which supplies pulses of modulated length (T) proportional to the said energy, with a period of recurrent (P) equal to that of the said component.

10. A device as claimed in claim 9, characterized in that the exciting circuit (18—28) comprises a current source which supplied a current of predetermined constant intensity for the modulated length (T) of the pulses.

11. A device as claimed in claim 8, characterized in that the output of the low-pass filter (108) is connected to a generator (107) which outputs pulses (T'1, T'2, T'3) of the same frequency as the said component and to a stage (130) coding the intensity of the current of the stimulation signal as a function of the output signal from the rectifying and filtering stage (106), the coding stage supplying the said control signal (Sc).

12. A device as claimed in claim 11, characterized in that the exciting circuit (118—123, 131) comprises a current source and a control-signal (Sc) decoding stage (131) which determines the intensity of the current (Is1, Is2, Is3) supplied to the stimulation electrode (118) by the current source for the length (T0) of said pulses (T'1, T'2, T'3).

13. A device as claimed in either one of claims 11 and 12, characterized in that the said pulses (T'1, T'2, T'3) have a constant length (T0).

14. A device as claimed in either one of claims 1 to 13, characterized in that the exciting circuit (18—23); 118—123; 131) and the stimulation electrode (18—118) form a first subassembly (2) implantable under the skin of a patient, the microphone (3) and the electronic processor (4—9; 104—109) forming a second subassembly (1) designed to be worn externally by the patient, and the transmission means (12—17; 112—117) comprises tuned receiving (16) and transmitting (14) antennas associated respectively with the first and second subassemblies.

EP 0 219 380 B1

FIG.:1

FIG.:2

FIG.:3

FIG.:4

FIG.: 5

FIG.:6

FIG.:9

2

EP 0 219 380 B1

FIG.:7

FIG.:8